# EUROPEAN PATENT APPLICATION

(11) **EP 4 803 598 A1**
(43) Date of publication of application: **09.09.2026**
(21) Application number: 25305285.6
(22) Date of filing: 03.03.2025
(51) Int. Cl.: C11B 1/02, C11B 1/10, C12N 1/00, C12P 7/6472

(54) **CRUDE OIL OF THRAUSTOCHYTRID RICH IN DOCOSAHEXAENOIC ACID AND EICOSAPENTAENOIC ACID**

(71) Applicant: Fermentalg, 33500 Libourne (FR)
(72) Inventor: NOGARO, Guillaume, 33500 Libourne (FR); RANGEL, Rodrigo, 33500 Libourne (FR); BOUHOUDA, Sammy, 33500 Libourne (FR); PEUMERY, Klara, 33500 Libourne (FR); DEMOL, Julien, 33500 Libourne (FR); GRIFFITHS, Hywel, 33500 Libourne (FR); COURTIAU, Emilie, 33500 Libourne (FR); COMMAULT, Audrey, 33500 Libourne (FR)
(74) Representative: Icosa

(57) **Abstract**

The present invention relates to a crude microbial oil of Thraustochytrid rich in docosahexaenoic acid (DHA) and eicosapentaenoic acid (EPA), which comprises at least 94% of triglycerides. The present invention further relates to a process for producing said crude microbial oil, as well as its uses.

## Description

### FIELD OF INVENTION

The present invention relates to a crude microbial oil of Thraustochytrid rich in docosahexaenoic acid (DHA) and eicosapentaenoic acid (EPA), which comprises at least 94% of triglycerides. The present invention further relates to a process for producing said crude microbial oil, as well as its uses.

### BACKGROUND OF INVENTION

Omega-3 fatty acids are Polyunsaturated Fatty Acids (PUFA) that are biologically important notably giving their effect on cellular physiology, gene expression of biologically active compounds, and their use as biosynthetic substrates.

For example, docosahexaenoic acid (DHA), accounts for approximately 15%-20% of lipids in the human cerebral cortex and 30%-60% of lipids in the retina; it is concentrated in the testes and sperm, and it is an important component of breast milk. Moreover, DHA is essential for both fetal and infant development as well as maintenance of cognitive functions in adults.

Because omega-3 fatty acids are not synthesized de novo in the human body, they must be derived from nutritional sources. For example, marine oils, oils extracted from fishes and/or microorganisms, are considered nutritionally good with regards to their omega-3 content, especially their DHA and/or eicosapentaenoic acid (EPA) contents.

However, fish oils vary considerably in composition and level of fatty acids depending on their particular species and their diets. For example, fish raised by aquaculture tend to have a lower level of omega-3 fatty acids than those in the wild. Furthermore, fish oils carry a risk of containing environmental contaminants.

Additionally, for a sustainable and environmentally friendly approach, fish consumption must be controlled, alternatives to fish meal have thus to be found and/or by-products of the fish meal industry have to be reduced and if any, reused.

The industry already uses fish meal by-products as starting material for producing modified oils rich in EPA and DHA. Such oils are produced thanks to concentration process steps like short-path distillation that generate unnatural, re-esterified triglycerides but also oxidized and contaminated by-products.

Some studies have demonstrated cytotoxic, neurotoxic, or even carcinogenic effects of oxidized omega-3 oils. Their roles in inflammation and impact on cholesterol levels have also been demonstrated. Experiments on this topic are however still limited in number and use animal models. Accordingly, there is not yet a clear picture of the actual impact of oxidized oils consumption on human health and a precautionary principle must be applied, thus currently such oils are discarded.

Oils derived from microorganisms have been identified as a possible alternative to omega-3 rich oils derived from fish. The fatty acid profiles of these microbial oils differ from the ones of fish oils and vary depending on the strain used. When searching for alternatives to fish oils, the food and feed industries are searching for oils with the greatest nutritional value, and which are most easily substituted into existing processes. To date, triglyceride oils from Thraustochytrid strains, in particular the genera *Schizochytrium* and *Aurantiochytrium* come closest to these requirements, but these are not yet a complete match in fatty acid profile.

There is thus a real need to produce microbial oils with fatty acids profiles closer to fish oils. For example, these microbial oils shall contain palmitoleic acid (PA, C16 :1), which is found in fish oils and which is of medical interest.

Therefore, a continuing need still exists for providing microbial oils with high productivity and desirable fatty acids profiles. More particularly, these oils shall be easy to produce at an industrial scale. They shall comprise high content of triglycerides and suitable contents of nutritionally good fatty acids, specifically EPA, DHA and PA. These oils shall also be safe for human consumption.

Thus, the present invention aims to provide a health-enhancing and more sustainable omega-3 rich microbial oil, in particular an oil rich in DHA and EPA that overcomes shortcomings of the prior art.

Surprisingly, the inventors have found that using specific marine oils, notably oxidized marine oils, as carbon source for growing Thraustochytrids strains allows to produce a crude non-oxidized microbial oil with a high content of triglycerides and an improved fatty acids profile including suitable contents of nutritionally good fatty acids specifically EPA, DHA and PA.

Further, advantageously, fatty acids of interest are directly and naturally found in triglyceride form within the microbial oil according to the invention without need to be subjected to chemical nor enzymatic process after their biosynthesis and extraction.

### SUMMARY

This invention thus relates to a crude microbial oil of at least one Thraustochytrid comprising:
- at least 94% of triglycerides relative to the total lipids,
- more than 5% by weight of eicosapentaenoic acid (EPA), relative to the total weight of oil,
- at least 27% by weight, preferably at least 30% by weight of docosahexaenoic acid (DHA), relative to the total weight of oil, and
- from 0.1% to 5% by weight of palmitoleic acid (PA), relative to the total weight of oil; wherein the p-anisidine value of said oil being lower than or equal to 20.

The present invention further relates to a process for producing a crude microbial oil of Thraustochytrid comprising the following steps:
a) culturing a Thraustochytrid strain within a culture medium in a reactor to form a biomass comprising an oil,
b) recovering the biomass formed at step a), and
c) extracting the oil from the biomass recovered at step b);
wherein, in step a), the thraustochytrid strain is cultured in presence of a marine oil comprising at least 50% by weight of free fatty acids, relative to the total weight of said marine oil, preferably an oxidized marine oil, more preferably an oxidized oil derived from fish.

Another object of the present invention relates to a crude microbial oil of Thraustochytrid obtained by the process according to the present invention, wherein the p-anisidine value of said oil being lower than or equal to 20, preferably lower than or equal to 15.

The present invention further relates to a biomass of Thraustochytrid comprising a crude microbial oil according to the present invention.

### DEFINITIONS

In the present invention, the following terms have the following meanings:
**"Biomass"** advantageously refers to microorganism cells produced through culture, in particular by methods as described above, and harvested, i.e., recovered from the fermentation broth, which comprises the cells and the culture medium, by separation of at least part of the culture medium.

**"Marine oil"** refers to an oil derived from fish and/or microorganisms.

**"Non-oxidized oils"** refers to marine oils having a p-anisidine value (also called para-Anisidine Value or pAV) lower than or equal to 20, preferably lower than or equal to 15. This value is analyzed on the oils according to the method 9.0 01/2018 of European Pharmacopoeia.

**"Oxidized marine oils"** refer to marine oils having anisidine value making them unfit for food consumption, in other words p-anisidine value higher than 20 preferably higher than 35. In particular, said oxidized marine oils are by-products from production of concentrated marine oils, preferably fish oils.

**"Omega-3 fatty acids",** also called **"n-3 fatty acids",** correspond to polyunsaturated fatty acids (PUFAs) comprising a double bond three atoms away from the terminal methyl group in their structure. Among omega-3 fatty acids, mention may be made of docosahexaenoic acid (DHA) and eicosapentaenoic acid (EPA). In the present description, omega-3 fatty acids are identified with an "n3" ending such as EPA: C20:5**n3** or DHA: C22:6**n3**.

A **"fatty acid"** corresponds to a hydrocarbon chain with a methyl and a carboxylic acid at either end, C₆-C₃₀, preferably a C₁₂-C₂₄ chain. It can be linear or branched. In addition to chain length, fatty acids differ from each other in number, type, and location of double bonds. Fatty acids can thus be saturated or unsaturated. They can notably be found in free form (i.e. free fatty acid or FFA), or esterified on a glycerol forming monoglycerides, diglycerides or triglycerides.

**"Fatty acids Ethyl Ester (EE)"** are fatty acids on which the glycerol backbone of a triglyceride is replaced with ethanol. Then, "re-esterified triglycerides" are created when EEs are freed from their ethanol backbone and are reunited with a glycerol molecule.

**"Reactor"** refers to a fermenter either at laboratory, pilot or industrial scale.

**"Crude oil"** refers to an oil that is extracted from a biomass of microorganisms without further processing, nor dilution with another oil like vegetable oil. Notably, it refers to an oil that is not refined and that has not been subjected to purification techniques known in the art, such as distillation or urea adduction, to produce a product with higher concentrations of DHA, of EPA or of another fatty acid.

**"Refined oil"** refers to an oil that is obtained by treating a crude oil with standard processes of refining.

**"About"** is used herein to mean approximately, roughly, around, or in the region of. The term "about" preceding a figure means more or less 10 % of the value of the figure. When the term "about" is used in conjunction with a numerical range, it modifies that range by extending the boundaries above and below the numerical values set forth by 10%.

**"At least"** is equivalent to the expression "greater than or equal to" when it refers to contents, or to the expression "one or more" when it refers to components.

**"Comprising"** or **"comprise"** is to be construed in an open, inclusive sense, but not limited to.

**"From X to Y"** refers to the range of values between X and Y, the limits X and Y being included in said range.

Lipids contents and profiles are analyzed by methods known by the skilled in the art. For example, as detailed below in the Examples. Notably, the fatty acids profiles of the oils are analyzed and expressed according to the European Pharmacopoeia method for omega-3 analysis.

### DETAILED DESCRIPTION

### The crude microbial oil

The present invention relates to a crude microbial oil of at least one Thraustochytrid (also called crude microbial oil of Thraustochytrid) comprising:
- at least about 94% of triglycerides relative to the total lipids,
- more than 5% by weight of eicosapentaenoic acid (EPA), relative to the total weight of oil,
- at least 27% by weight, preferably at least about 30% by weight of docosahexaenoic acid (DHA), relative to the total weight of oil, and
- from about 0.1% to about 5% by weight of palmitoleic acid (PA), relative to the total weight of oil;
wherein the p-anisidine value of said oil being lower than or equal to 20.

Advantageously, the at least one Thraustochytrid may be of the order Thraustochytriales, preferentially of the subclass Thraustochytriaceae, more preferentially of a genus which may be selected from the group comprising the genera *Aurantiochytrium, Aplanochytrium, Botryochytrium, Japonochytrium, Oblongichytrium, Parietichytrium, Schizochytrium, Sicyoidochytrium, Thraustochytrium, Monorhizochytrium* and *Ulkenia.* The one skilled in the art understands that this definition encompasses all microorganisms currently classified within these groups, irrespective of future phylogenetic revisions.

Advantageously, the at least one Thraustochytrid is selected from the strains of the genera *Aurantiochytrium* and *Schizochytrium.* Preferably, the at least one Thraustochytrid is selected from the species of *Aurantiochytrium mangrovei, Aurantiochytrium Hondaea, Schizochytrium limacinum, Schizochytrium aggregatum, Schizochytrium sp.* and *Aurantiochytrium sp.*

More preferably, the Thraustochytrids are selected from the strains of *Aurantiochytrium mangrovei* species and *Schizochytrium limacinum* species.

Preferably, the at least one Thraustochytrid is selected from the strains *Aurantiochytrium mangrovei* CCAP 4062/7 (deposited before the International Depositary Authority Culture Collection of Algae and Protozoa, UK, on the 6 September *2018); Aurantiochytrium mangrovei* CCAP 4062/2; *Aurantiochytrium mangrovei* CCAP 4062/3 (CCAP4062/3); *Aurantiochytrium mangrovei* CCAP 4062/4; *Aurantiochytrium mangrovei* CCAP 4062/5; *Aurantiochytrium mangrovei* CCAP 4062/6; *Aurantiochytrium mangrovei* CCAP 4062/1; *Schizochytrium limacinum SR21* (ATCC MYA-1381); more preferably selected from the strains *Aurantiochytrium mangrovei* CCAP4062/7 and CCAP4062/3 and *Schizochytrium limacinum SR21.*

Advantageously, the at least one Thraustochytrid used in the present invention is non-genetically modified microorganisms. If genetically modified Thraustochytrids are used, they do not contain genes encoding for one or more enzymes that make it possible to modify the oil produced by the strain.

The crude microbial oil according to the present invention comprises essentially fatty acids in the form of triglycerides. The content of triglycerides, present in the crude microbial oil of the invention, represents at least 94%, preferably from 96% to 99% relative to the total lipids present in the oil. The triglyceride content is for example analyzed by gas phase chromatography. The triglyceride content is expressed in relative percentage to the sum of the lipids measured in the oil, i.e. in relative percentage to the total mass of fat within the oil.

Advantageously, the crude microbial oil according to the present invention comprises at least 35% by weight of omega-3 fatty acids, relative to the total weight of oil. Preferably, the content of omega-3 fatty acids, present in the crude microbial oil of the invention ranges from 35% to 90 % by weight, still preferably from 36% to 50% by weight, relative to the total weight of oil.

The crude microbial oil of Thraustochytrid of the present invention comprises more than 5% of eicosapentaenoic acid (EPA), relative to the total fatty acids content.

Advantageously, the amount of EPA, present among the fatty acids (also referred as the fatty acids fraction) of the crude microbial oil according to the invention, ranges from 5% to 30%, preferably from 7% to 20%, relative to the total fatty acids content.

Advantageously, the amount of EPA, present among the triglycerides (also referred as the triglycerides fraction) of the crude microbial oil according to the invention, ranges from 5% to 30%, preferably from 7% to 20%, relative to the total triglycerides content.

Advantageously, the amount of EPA, present in the crude microbial oil according to the invention, is greater than 5% by weight, preferably ranges from 5% to 30% by weight, still preferably from 7% to 20% by weight, relative to the total weight of oil.

The crude microbial oil of thraustochytrid of the present invention further comprises at least 27%, preferably at least 30% of docosahexaenoic acid (DHA), relative to the total fatty acids content.

Advantageously, the amount of DHA, present among the fatty acids (also referred as the fatty acids fraction) of the crude microbial oil according to the invention, ranges from 30% to 60%, preferably from 40% to 50%, relative to the total fatty acids content.

Advantageously, the amount of DHA, present among the triglycerides (also referred as the triglycerides fraction) of the crude microbial oil according to the invention, is greater than or equal to 27%, preferably it ranges from 30% to 60%, more preferably from 40% to 50%, relative to the total triglycerides content.

Advantageously, the amount of DHA, present in the crude microbial oil according to the invention, is greater than or equal to 27% by weight, preferably greater than or equal to 30% by weight, more preferably it ranges from 30% to 60% by weight, still preferably from 40% to 50% by weight, relative to the total weight of oil.

Advantageously, the amount of PA, present among the fatty acids (also referred as the fatty acids fraction) of the crude microbial oil according to the invention, ranges from 0.1% to 5%, preferably from 1% to 4%, relative to the total fatty acids content.

Advantageously, the amount of PA, present among the triglycerides (also referred as the triglycerides fraction) of the crude microbial oil according to the invention, ranges from 0.1% to 5%, preferably from 1% to 4%, relative to the total triglycerides content.

Advantageously, the amount of PA, present in the crude microbial oil according to the invention, is greater than or equal to 0.1% by weight, preferably ranges from 0.1% to 5% by weight, still preferably ranges from 1% to 4% by weight, relative to the total weight of oil.

Advantageously, the crude microbial oil of Thraustochytrid of the present invention may further comprise linoleic acid (sum of all C18:2 fatty acids) in an amount greater than or equal to 0.1% by weight, preferably ranging from 0.1% to 2%, relative to the total weight of oil.

Advantageously, the crude microbial oil of Thraustochytrid of the present invention may further comprise linoleic acid (sum of all C18:2 fatty acids) in an amount greater than or equal to 0.1%, preferably ranging from 0.1% to 2%, relative to the total fatty acids content.

Advantageously, the crude microbial oil of Thraustochytrid of the present invention may further comprise oleic acid (C18:1) in an amount ranging from 0.1% to 10% by weight, preferably ranging from 1% to 8% by weight, relative to the total weight of oil.

Advantageously, the crude microbial oil of Thraustochytrid of the present invention may further comprise oleic acid (C18:1) in an amount ranging from 0.1% to 10%, preferably ranging from 1% to 8%, relative to the total fatty acid content.

Advantageously, the crude microbial oil of Thraustochytrid of the present invention is free from re-esterified triglycerides. In other words, the crude microbial oil of Thraustochytrid of the present invention advantageously comprises less than 0.05%, preferably less than 0.01%, and more preferably less than the limit of detection or 0% of re-esterified triglycerides, relative to the oil.

Advantageously, the crude microbial oil of Thraustochytrid of the present invention is free from solvents (such as ethanol or methanol). In other words, the crude microbial oil of Thraustochytrid of the present invention advantageously comprises less than 0.05% by weight, preferably less than 0.01% by weight, and more preferably 0% by weight of solvent, relative to the total weight of the oil.

The crude microbial oil of Thraustochytrid of the present invention is non-oxidized., i.e. p-anisidine value (or para-Anisidine Value (pAV)) of the oil being lower than or equal to 20, preferably lower than or equal to 15.

Advantageously, the peroxide value (PV) of the crude microbial oil of thraustochytrid of the present invention is lower than or equal to 5 mEqO₂/kg, preferably ranges from 0.01 to 5 mEqO₂/kg.

### The process for production the crude microbial oil

The present invention further relates to a process for producing a crude microbial oil of Thraustochytrid comprising the following steps:
a) culturing a Thraustochytrid strain within a culture medium in a reactor to form a biomass comprising an oil,
b) recovering the biomass formed at step a), and
c) extracting the oil from the biomass recovered at step b);
wherein, in step a), the Thraustochytrid strain is cultured in presence of a marine oil comprising at least 50% by weight of free fatty acids, relative to the total weight of the marine oil.

### Step a)

The process of the present invention comprises a step a) of culturing a Thraustochytrid strain within a culture medium in a reactor to form a biomass comprising an oil. During this step a), the Thraustochytrid strain is cultured in the presence of a marine oil comprising at least 50% by weight of free fatty acids, relative to the total weight of the marine oil.

The inventors have surprisingly found that the presence of marine oil, preferably comprising at least 50% by weight of free fatty acids, relative to the total weight of the marine oil, during the culture of a Thraustochytrid strain according to the invention allows significant growth of Thraustochytrid biomass with high oil content, exhibiting a suitable fatty acid profile.

Advantageously, step a) of culturing the Thraustochytrid strain is carried out in a culture medium and under conditions suitable for promoting growth of the strain and lipid accumulation.

The skilled in the art will be able to adapt culture conditions as described in WO2012/035262, WO2015/004402 and WO2015/004403. The skilled person will be notably able to adapt culture medium composition, conditions for adding nutrients during cultivation, temperature cycle, oxygenation and lighting.

There are three types of fermentation processes commonly used in the field of biomass production, any of which, or modifications thereof, may be applied to carry out the present invention.:
- batch mode: the reactor is filled with the culture medium, then the strain is added at a suitable inoculation percentage (v/v) and cultivation then takes place without any major additional addition of medium (with corrections nonetheless being made for pH, levels of aeration and agitation, and to control foaming);
- fed-batch mode: this production process starts as per batch mode but includes a subsequent feeding of nutrient substrate during cultivation that may be either gradual or in defined volumes; and
- continuous mode: fresh culture medium is continuously added in the reactor, while culture liquid is continuously withdrawn to keep the culture volume constant; a variant upon continuous mode has defined volumes of culture withdrawn periodically and replaced with fresh medium.

According to a preferred embodiment, step a) of culturing at least one Thraustochytrid strain is carried out using the fed-batch culture technique, which allows the carbon source to be maintained at non-inhibitory concentrations, while promoting an increase in biomass.

Advantageously, step a) of culturing a Thraustochytrid strain is carried out under heterotrophic or mixotrophic conditions, preferably heterotrophic.

Advantageously, step a) of culturing a Thraustochytrid strain is carried out with at least one organic carbon source such as a sugar, an alcohol, an organic acid or its salt, a fat (for example an oil) and/or amino acids and proteins. In this case, the carbon source can be selected from glucose or dextrose, fructose, saccharose, maltose, glycerol, acetic acid, starch and/or oils.

Preferably, the suitable culture medium to which the marine oil is added is a medium that comprises a carbon source, a nitrogen source, a phosphorus source and salts.

Preferably the marine oil is an oxidized marine oil, and more preferably an oxidized oil derived from fish.

Advantageously, the marine oil used in step a) comprises at least 50 % by weight of free fatty acids, preferably from about 50% to 99% by weight, still preferably from about 60% to 95% by weight, relative to the total weight of the marine oil.

Advantageously, the marine oil used in step a) comprises at least 5%, preferably from 5% to 55%, still preferably from 8% to 40% of EPA in its free fatty acid form, relative to the total fatty acids content of the marine oil.

Advantageously, the marine oil used in step a) comprises at least 1%, preferably from 1% to 20%, still preferably from 5% to 15% of DHA in its free fatty acid form, relative to the total fatty acids content of the marine oil.

Advantageously, the marine oil used in step a) comprises at least 0. 1%, preferably from 0.1% to 60%, still preferably from 10% to 50% of PA in its free fatty acid form, relative to the total fatty acids content of the marine oil.

Advantageously, the marine oil used in the process of the present invention is an oil derived from fish; preferably chosen among by-products of fish oil production, said by-products comprising at least 50% by weight of free fatty acids, relative to the total weight of by-products. More preferably, the marine oil is a distillate of free fatty acids ("FFA distillate") obtained through distillation of a fish oil notably short path distillation or an acid oil obtained through saponification of a fish oil.

According to the invention, the culturing step a) comprises addition of the marine oil into the reactor either before or after inoculation of the strain.

According to the invention, the culturing step a) comprises the use of a culture medium comprising or not another carbon source than the present marine oil, for example glucose.

According to a first embodiment, the marine oil is present in the initial culture medium of the culturing step a).

According to another embodiment, the marine oil is added in the culture medium, preferably comprising glucose. In other word, the culture medium is added first to the reactor and the marine oil is directly added to the medium. According to this embodiment, the marine oil is present in the culture medium.

According to a first variant of this embodiment, the culture medium further comprises glucose. In other words, according to this variant, the Thraustochytrid strain is cultured in presence of both marine oil and another carbon source, preferably glucose, sequentially and/or simultaneously.

According to a second variant of this embodiment, the carbon source is the marine oil only. In other words, according to this variant, the culture medium is free of any other carbon source according to the invention, in particular free of glucose.

According to still another embodiment, the marine oil is added in the culture medium during the culturing step a), preferably after at least about 5 hours of culture, more preferably after about 15 hours of culture, still more preferably after at least about 45 hours of culture.

According to a first variant of this embodiment, the marine oil is added after nitrogen starvation of the biomass. According to the invention, **"nitrogen starvation"** means a condition where the cultivated strain is limited in its access to nitrogen sources and in particular a complete depletion of nitrogen presents in the media as metabolically available nitrogen.

According to another variant of this embodiment, the marine oil is added, during the culturing step a), directly to the fermentation broth (which comprises the cells and the culture medium), without nitrogen starvation.

### Step b)

The process of the present invention further comprises a step b) of recovering the biomass formed at step a).

As part of the recovery process the physical integrity of the biomass cells may be degraded incidentally to the process. It is therefore understood that said biomass may include a quantity of degraded microbial cells from 0% to 100%, preferably from 0% to 50%. "Degraded" means that the physical integrity of said microorganism cells may have been altered, such as lysed microorganisms, resulting for example from a process of homogenization or enzymatic lysis. Once produced, this biomass can be raw, just separated from its culture medium, dried or not, degraded or not.

The biomass, depending on whether it is dried or not, totally or partially, can have a moisture content of 1% to 90%.

The invention therefore also relates to a biomass of microorganisms comprising an oil as previously defined.

According to a first embodiment, the biomass has a moisture content of 70% to 90%, preferentially 80% to 85%. This is particularly the case of the biomass harvested by filtration of the fermentation broth that is not or partially dried.

According to another embodiment of the invention, the biomass is dried, totally or partially, and has a moisture content of 1% to 10%, preferentially 2% to 7%.

The biomass may be packaged for storage or for use as such, for example as a food supplement or food for human or animal consumption.

Advantageously, before extraction step c), the biomass is washed in order to remove any remaining exogenous marine oil present in culture step a).

This washing can be done by any means known by the one skilled in the art and as much time as needed to obtain a biomass without exogenous marine oil residues. In particular, the biomass can be washed by dilution into water, centrifugation and discarding of the supernatant comprising oils residues.

### Step c)

The process of the present invention further comprises a step c) of extracting the oil from the biomass recovered at step b).

The methods for isolating or extracting an oil according to the invention from a biomass produced by the culture of microorganism cells are well known to the person skilled in the art. Particular mention may be made of solid-liquid extraction which is based on the use of a solvent (liquid phase) to extract the oil contained in the dried biomass (solid phase) by sprinkling or maceration; liquid-liquid extraction which is based on the separation of the aqueous phase from the oil after preliminary lysis of the cells and then decanting or centrifugation. Preferably the extraction is done without organic solvents. Particular mention may be made of the applications WO 01/53512, WO 02/10423, WO 2014/122092, WO 2015/092546 and WO 2015/095694.

Mention may also be made of a method for improving the fat extraction yields from microorganisms for PUFA-rich oils. This method consists in carrying out cell lysis at a first temperature, the latter being continued at a second temperature lower than the first, then mechanical separation of the oil from the lysed biomass (filtration, decanting) (WO2020/053375).

The mechanical separation of an oil from a lysed biomass is also well known to the skilled person, as a gravity separation, in particular by centrifugation as described in patent application WO 01/53512. It is also possible to use continuous separation, in particular by centrifugal plate separator. Such separators are known to continuously extract oils from complex media comprising solid residues and water, as described in patent application WO 20101096002, in particular marketed by the companies Alfa Laval, Flottweg or SPX Flow Technology Santorso, among others. This continuous separation step is preferred in the process used to obtain the oil according to the invention.

Advantageously, the process of the present invention comprises no further step of treatment of the crude oil extracted in step c). Preferably, the process of the invention does not comprise any refining step of the crude oil extracted in step c).

The microbial oil obtained at the end of the process is a crude oil, preferably a non-oxidized crude oil, i.e. the p-anisidine value of the crude oil being lower than or equal to 20, preferably lower than or equal to 15.

Advantageously, the peroxide value of microbial oil obtained at the end of the process is lower than 5 mEqO₂/kg, preferably ranges from 0.01 to 5 mEqO₂/kg.

The present invention further relates to a crude microbial oil of Thraustochytrid obtained by the process of the present invention, wherein the p-anisidine value of said oil being lower than or equal to 20, preferably lower than or equal to 15.

The present invention also relates to a biomass of Thraustochytrid comprising the crude microbial oil of the present invention.

The present invention further relates to a biomass of Thraustochytrid obtained by the process of the present invention.

The present invention also relates to the use of a crude microbial oil according to the invention for human or animal food, in particular food for petfood, feed notably aquaculture or pharmaceutical applications.

All the features described above for the crude microbial oil (part "The crude microbial oil") apply *mutatis mutandis* to the process for producing said crude microbial oil, as well as to the biomass and uses thereof.

### BRIEF DESCRIPTION OF THE DRAWINGS

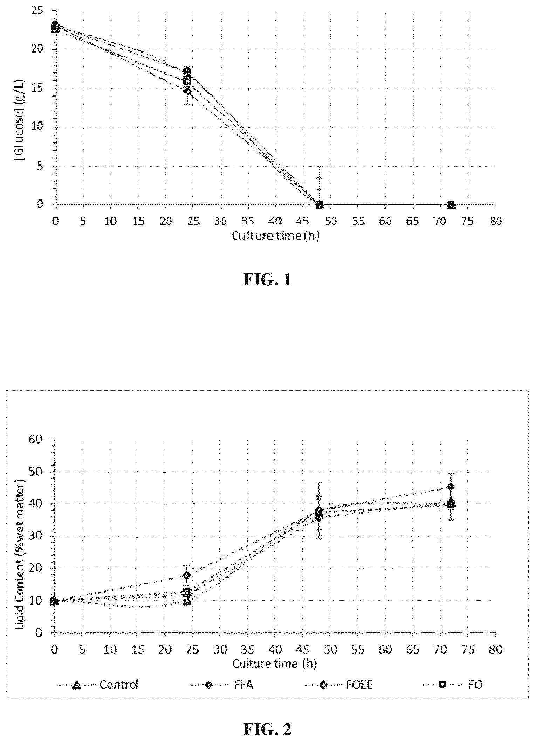

**Figure 1** represents a graph showing the evolution of glucose consumption by *Aurantiochytrium mangrovei* strain CCAP4062/7during culture in absence (control) or presence of different oils (FFA by product (FFA), Fish oil ethyl ester (FOEE) or crude fish oil (FO)).
**Figure 2** represents a graph showing the evolution of lipids production by *Aurantiochytrium mangrovei* strain CCAP4062/7during culture in absence (control) or presence of different oils (FFA by product (FFA), Fish oil ethyl ester (FOEE) or crude fish oil (FO)).

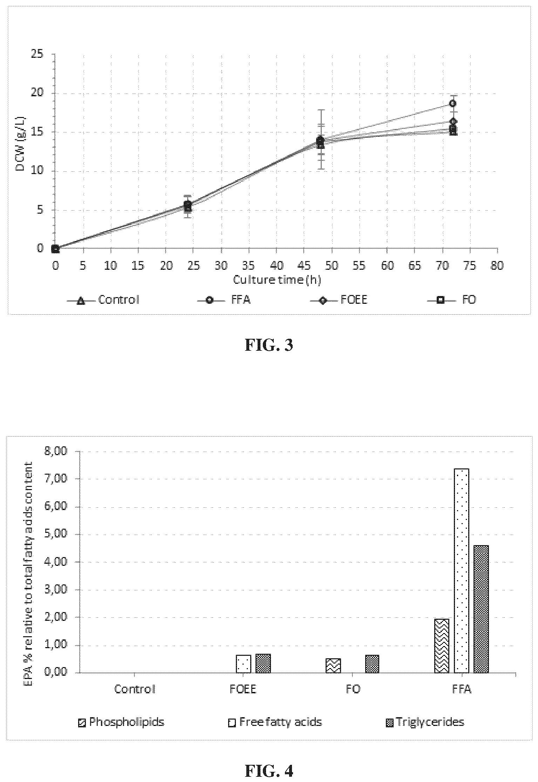

**Figure 3** represents a graph showing the biomass dry cell weight, representing the amount of biomass produced during culture of *Aurantiochytrium mangrovei* strain CCAP4062/7in absence (control) or presence of different oils used as carbon source (FFA by product (FFA), Fish oil ethyl ester (FOEE) or crude fish oil (FO)).
**Figure 4** represents a histogram showing the distribution of eicosapentaenoic acid (EPA) in the lipids produced by *Aurantiochytrium mangrovei* strain CCAP4062/7 after culture in absence (control) or presence of different oils used as carbon source (FFA by product (FFA), Fish oil ethyl ester (FOEE) or crude fish oil (FO)). The contents of EPA have been measured in three distinct lipidic fractions present in the lipids: phospholipids, free fatty acids and triglycerides. They are expressed in %, relative to the total lipidic fraction content containing EPA.

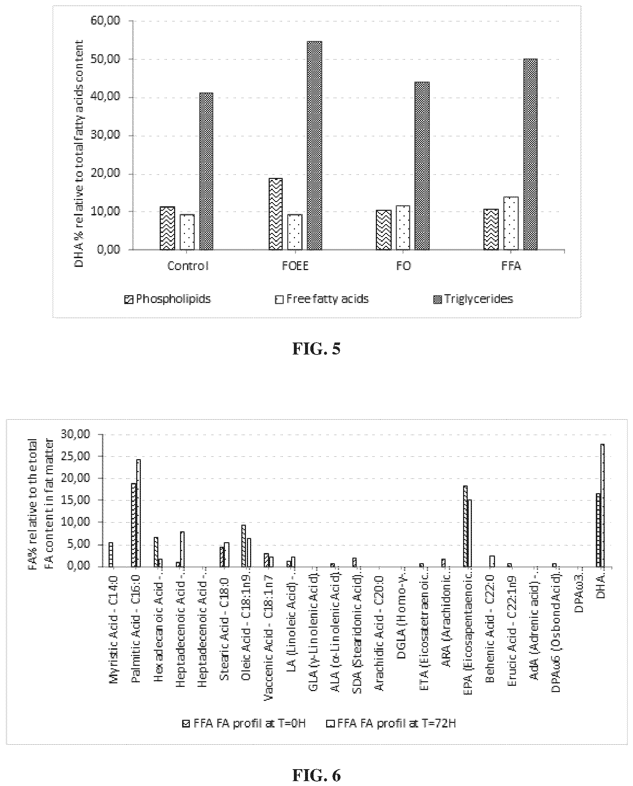

**Figure 5** represents a histogram showing the distribution of docosahexaenoic acid (DHA) in the oils produced by *Aurantiochytrium mangrovei* strain CCAP4062/7 after culture in absence (control) or presence of different oils used as carbon source (FFA by product (FFA), Fish oil ethyl ester (FOEE) or crude fish oil (FO)). The contents of DHA have been measured in three distinct lipidic fractions present in the oils: phospholipids, free fatty acids and triglycerides. They are expressed in %, relative to the total fatty acids content of the lipidic fraction containing DHA.
**Figure 6** represents the fatty acid profile of the lipidic phase of the culture medium supplemented with FFA distillate at t = 0 and 72 hours after culture of *Aurantiochytrium mangrovei* strain CCAP4062/7. The contents of fatty acids are expressed in percentage, relative to the total fatty acid content of the lipidic phase.

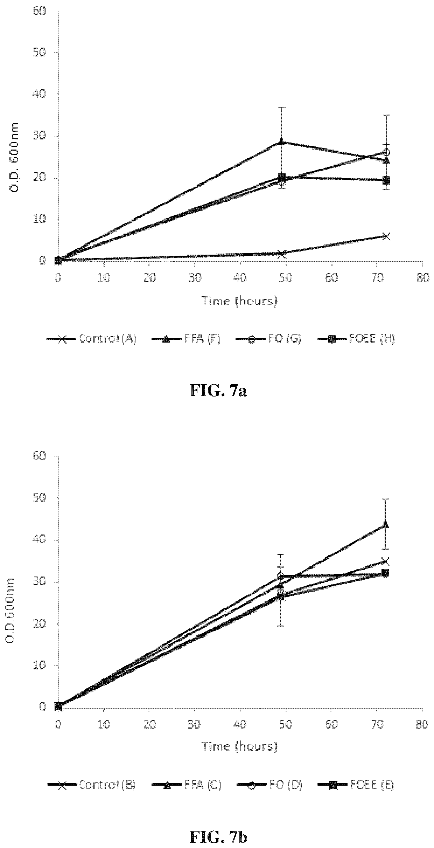

**Figures 7a** and **7b** represent two graphs showing the optical densities (600 nm) of the biomasses of *Aurantiochytrium mangrovei* strain CCAP4062/7 cultivated in absence (control) or presence of different oils used as carbon source (FFA by product (FFA), Fish oil ethyl ester (FOEE) or crude fish oil (FO)); the media being either supplemented with glucose **(7b)** or not **(7a).**

### EXAMPLES

The present invention is further illustrated by the following examples.

### Materials and Methods

### Strains

The five following strains have been used in the examples:
- *Aurantiochytrium mangrovei* (deposited before the International Depositary Authority Culture Collection of Algae and Protozoa, UK, on the 6 September 2018 with the accession number CCAP 4062/7);
- *Aurantiochytrium mangrovei* (CCAP4062/3, CCAP 4062/3);
- *Schizochytrium limacinum SR21* (ATCC MYA-1381);
- *Auxenochlorella protothecoides* (CCAP 211-8D); and
- *Nitzschia laevis* (UTEX2047).

### Culture media

Each strain was cultivated in a growth medium suitable for its genus and species. In particular, both *Aurantiochytrium mangrovei* strains were cultivated in a medium as presented in the following Table *1; Schizochytrium limacinum SR21* in commercial ATCC 790/30 medium; *Auxenochlorella protothecoides* in commercial UTEX Euglena medium; and *Nitzschia laevis* in a medium as presented in Table 2.

**Table 1**

| Growth medium for *Aurantiochytrium mangrovei* strains | | |
|---|---|---|
| CaCl₂, 2H₂O | 0.55 | g/L |
| MgSO₄, 7H₂O | 4.01 | g/L |
| H₃BO₃ | 0.00875 | g/L |
| KH₂PO₄ | 4 | g/L |
| Na₄EDTA, 2H₂O | 0.12 | g/L |
| FeSO₄, 7H₂O | 0.04 | g/L |
| (NH₄)₂SO₄ | 1 | g/L |
| MnCl₂, 4H₂O | 0.0108 | g/L |
| ZnSO₄, 7H₂O | 0.0108 | g/L |
| CoCl₂, 6H₂O | 0.000108 | g/L |
| Na₂MoO₄, 2H₂O | 0.000108 | g/L |
| Na₂SeO₃ | 1.73E-07 | g/L |
| CuSO₄, 5H₂O | 0.0072 | g/L |
| Thiamine | 0.032 | g/L |
| Vitamine B12 | 0.0005 | g/L |
| Panthotenate | 0.0108 | g/L |
| Antimousse Biospumex 153K | 0.4 | mL/L |

**Table 2**

| Growth medium for *Nitzschia laevis* strain | | |
|---|---|---|
| NaCl | 8 | g/L |
| MgSO₄, 7H₂O | 2.2 | g/L |
| KCl | 0.75 | g/L |
| Tris | 0.8856 | g/L |
| CaCl₂, 2H₂O | 0.1 | g/L |
| FeCl₃, 6H₂O | 0.002376 | g/L |
| Citrate | 0.22 | g/L |
| ZnCl₂ | 0.0006966 | g/L |
| CoCl₂, 6H₂O | 0.00005152 | g/L |
| MnCl₂, 4H₂O | 0.00056 | g/L |
| Na₂MoO₄, 2H₂O | 0.001436 | g/L |
| H₃BO₃ | 0.0685 | g/L |
| Na₂HPO₄, 12 H₂O | 1.51 | g/L |
| NaNO₃ | 6.17 | g/L |
| Na₂SiO₃ | 0.4 | g/L |
| CuSO₄ 5H₂O | 0.00003136 | g/L |

### Carbon sources

When used, glucose was added to the above-mentioned culture media depending on the nature of the strains:
- 30g/L of culture medium for both *Aurantiochytrium mangrovei* strains and *Schizochytrium limacinum SR21;*
- 22g/L of culture media for *Auxenochlorella protothecoides* and
- 44g/L of culture media for *Nitzschia laevis.*

Furthermore, different oils were used either as an additional carbon source, or as a replacement for glucose or after glucose:
- a by-product oil from production of concentrated fish oil by distillation rich in FFA (referred to as FFA by product or FFA distillate);
- a crude fish oil (referred to as FO; GC Rieber);
- EPA ethyl esters (referred to as EPA-EE; Jiangsu Hainekon Biotechnology Co., Ltd. derived from Nannochloropsis);
- a palmitic-rich oil (KD Pharma);
- a refined fish oil comprising around 95% of FA ethyl esters and less than 1% of FFA (referred to as FOEE or esterified fish oil; GC Rieber);
- High Oleic Sunflower Oil (referred as HOSO; Jules Brochenin.);
- Olive oil (Cooking and seasoning oil);
- Soybean oil (Sigma);
- Colza oil (Cooking and seasoning oil) and
- Flaxseed oil (Cooking and seasoning oil).

The glyceridic profiles of the FFA distillate, the crude fish oil (FO crude) and the EPA ethyl esters (EPA-EE) are expressed in the Table 3 here below.

**Table 3**

| | **FFA distillate** | **EPA-EE** | **FO crude** |
|---|---|---|---|
| Glycerol | <0.1 | <0.1 | <0.1 |
| FFA | 63.6 | 0.4 | 5.5 |
| FA esters | 14.7 | 98.4 | - |
| Monoglycerides | 1.1 | 0.3 | 0.6 |
| Sterols | 2.5 | <0.1 | 0.6 |
| Diglycerides | 2 | <0.1 | 3.2 |
| Sterols esters | <0.1 | <0.1 | <0.1 |
| Triglycerides | 12.3 | <0.1 | 89.9 |
| Not identified | 3.8 | 0.8 | 0.2 |

### Lipid analyses

Several lipid analyses were done on the studied samples: fatty acids (FA) profiles of the biomasses and of the extracted crude oils, glyceridic profile and lipid content (over wet or dry matter). Given the fact that some of the tested biomasses were cultivated in the presence of marine or other oils, cells were washed before analysis, or when applicable before oil extraction, in order to avoid interferences on said lipids analysis results. The washing step implies centrifugation of the recovered biomass at a centrifugation speed enough to separate the liquid phase from the solid phase, the supernatant (or liquid phase) is discarded, and the biomass is resuspended with a volume equal to the discarded volume of softened water. This is repeated twice.

Fatty Acids (FA) profiles: For analyzing the FA profiles of the biomass, the samples were subjected to transmethylation using methanol, acid, and heat (80°C) to convert fatty acids into fatty acid methyl esters (FAMEs). After extraction with heptane, the FAMEs were analyzed by gas chromatography-FID. FA content was calculated as a percentage of total FAMEs according to the area under the curve.

The Fatty Acids (FA) profiles of the oils are analyzed according to the European Pharmacopoeia method for omega-3 analysis.

Glyceridic profiles: Oils were extracted from recovered biomass using the Folch method. The extracted oils were analyzed using gas phase chromatography to determine free fatty acid, monoglyceride, diglyceride and triglyceride (%) in the oil. The % of free fatty acid, monoglyceride, diglyceride and triglyceride correspond to relative percentage to the sum of lipids measured in the oil, i.e. relative percentage to the total mass of fat.

In some cases, the oils were extracted thanks to Folch method, then fractionated through a Solid Phase Extraction column. The samples were then prepared according to a method NF EN ISO 12966-2 and analyzed according to NF EN ISO 12966-4 by gas phase chromatography of fatty acid methyl esters (FAME). The results are given in % of total fatty acid content in the fraction.

Lipid content: Lipid content refers to the fat matter weight either over the wet matter weight, i.e. the weight of the wet biomass (%), or over the dry matter weight, ie. the weight of the freeze-dried biomass. Lipid content of the sample is calculated as follow: $Lipid content = \frac{weight of lipid \left(mg\right) \times 100}{wet or freeze - dried biomass weight \left(g\right)}$

Regarding the lipid content (%wet matter), the method comprises the following steps: The pH of the fermentation broth was adjusted between 8 and 8.6 with a NaOH solution at 0.1mol/L. The sample was then enzymatically lysed (Novozym 37071) in a water bath at 65°C under stirring for 30 minutes then 100°C for 10 minutes to obtain a wet biomass sample. 20mL of ethanol:heptane (1:2) was added in a sample of 2.5g of wet biomass in a 50mL Falcon and homogenized. Then, 1mL of KCl 5% was added and homogenized. The sample was then centrifuged 15 minutes at 3000rpm (Eppendorf Centrifuge 5424) and the organic phase recovered. A second washing was then made on the sample with 10mL of heptane. The sample was centrifuged again, the organic phase recovered then evaporated and finally weighted giving the weight of lipids in the sample.

Regarding the lipid content (%dry matter), the method comprises the following steps: After crushing the freeze-dried biomass, it was extracted with 1mL of chloroform:methanol (2:1) and homogenized in Tissuelyser at 30 Hz for 3 minutes. Then 200µL of 0.9% NaCl was added and homogenized. The sample was then centrifuged at 13000rpm for 5 minutes and organic phase recovered. The supernatant was washed again but with 1mL of chloroform and organic phase recovered and added to the previous one. The organic phases mix thus obtain was dry evaporated under nitrogen in a dry bath at 50°C and finally weighted giving the weight of lipids in the sample.

Residual glucose analysis: The residual glucose content is determined by biochemical analysis, in particular using a YSI^{®} biochemical analyzer following the manufacturer's recommendations.

Dry Cell Weight (DCW): The whole fermentation broth was recovered. Said broth is vacuum filtered then dried into a thermobalance and weighted.

Para-Anisidine Value (pAV): This value is analyzed on the oils according to the method 9.0 01/2018 of European Pharmacopoeia.

Peroxide value (PV): This value is analyzed on the oils according to the method NF EN ISO 3960.

### EXAMPLE 1: Comparison of oils produced with different microorganisms in presence of free fatty acids (FFA) distillate as carbon source

Four strains of different genus and/or species were cultivated in presence or absence of free fatty acids (FFA) distillate as a carbon source (*Aurantiochytrium mangrovei CCAP4062*/*3, Schizochytrium limacinum SR21, Auxenochlorella protothecoides* and *Nitzschia*)*.*

Each strain was inoculated at a defined percentage (1%v/v for *Aurantiochytrium mangrovei* CCAP4062/3and *Schizochytrium limacinum SR21;* 5%v/v for *Auxenochlorella protothecoides* and 10%v/v for *Nitzschia* relative to the culture medium volume) in the suitable medium comprising glucose as described hereabove, in Erlenmeyer flasks 250mL, in heterotrophy at 140 rpm with an orbital of 25mm. Immediately after, 1% v/v of FFA distillate with respect of the total volume of the fermentation broth was added in the flask. The culture time is: 72h at 30°C for *Aurantiochytrium mangrovei* CCAP4062/3and *Schizochytrium limacinum SR21;* 96h for *Auxenochlorella protothecoides* and *Nitzschia* respectively at 26°C and 22°C.

At the end of the culture time, the cells were harvested in a 50mL tube and centrifuged at 4500rpm at 4°C for 3 minutes. The supernatant was discarded, and the inner wall of the tube was wiped with a paper towel to remove the residue of added oils. Then, the pellet was resuspended and centrifuged again in the same conditions. The supernatant was discarded, and the pellet was resuspended at the same volume as the harvested fermentation broth. Analysis of the dry cell weight (DCW) and lipid content (%dry matter) were performed according to the methods described hereabove.

The results thus obtained are presented in the following Table 4.

| | *Aurantiochytrium mangrovei* CCAP4062/3 | | *Schizochytrium limacinum SR21* | | *Auxenochlorella protothecoides* | | *Nitzschia* | |
|---|---|---|---|---|---|---|---|---|
| | DCW (g/L) | Lipid content (%) | DCW (g/L) | Lipid content (%) | DCW (g/L) | Lipid content (%) | DCW (g/L) | Lipid content (%) |
| Control (without FFA distillate) | 18.05 | 44.15 | 2.50 | 56.58 | 11.83 | 29.13 | 9.32 | 14.87 |
| With FFA distillate | 21.93 | 49.65 | 1.75 | 65.75 | 13.70 | 42.25 | - | - |

The results thus obtained show that zero growth (corresponding to DCW values) was observed for *Nitszchia* when FFA distillate was added in the culture medium whereas a growth was observed within the culture medium without FFA distillate. In comparison, a growth was observed for *Aurantiochytrium mangrovei CCAP4062*/*3, Schizochytrium limacinum SR21* and *Auxenochlorella protothecoides* in both presence and absence of FFA distillate.

Thus, these results show that FFA by product can be used as alternative carbon source for tested strains other than *Nitszchia.* In addition, for *Aurantiochytrium mangrovei* CCAP4062/3and *Auxenochlorella protothecoides,* the biomass production is even increased in presence of FFA distillate in comparison with the use of glucose alone.

The results also show that the presence of FFA distillate in the fermentation medium improves the lipid content, and thus the fatty acids content, within the produced biomass in comparison with the use of glucose alone.

### EXAMPLE 2: Comparison of the oils produced with Aurantiochytrium mangrovei strains in presence of different oils as carbon source

Two *Aurantiochytrium mangrovei* strains (CCAP4062/7 and CCAP4062/3) were cultivated as the *Aurantiochytrium mangrovei* strain described in Example 1 but at 26°C and with different oils as additional carbon source (1% v/v of each tested oil with respect of the total weight of the fermentation broth). At the end of the culture, the cells were harvested a described in Example 1. Then, the fatty acids profiles of each produced oils were analyzed.

The results thus obtained are presented in Tables 5 and 6.

**Table 5: Results obtained for Aurantiochytrium mangrovei CCAP4062/7**

| | *Aurantiochytrium mangrovei* CCAP4062/7 | | | | | | | | | | | |
|---|---|---|---|---|---|---|---|---|---|---|---|---|
| | Control | Colza | FFA distillate | FOEE | FO 18% | FO crude | HOSO | Olive | Soybean | Walnut | EPA-EE | palmitic acid-rich oil |
| C12:0 | 0.00 | 0.00 | 0.00 | 0.00 | 0.00 | 0.00 | 0.00 | 0.00 | 0.00 | 0.00 | 0.00 | 0.00 |
| C14:0 | 0.30 | 0.26 | 0.87 | 0.77 | 0.74 | 0.62 | 0.25 | 0.25 | 0.26 | 0.26 | 0.27 | 0.75 |
| C14:1 | 0.00 | 0.00 | 0.00 | 0.00 | 0.00 | 0.00 | 0.00 | 0.00 | 0.00 | 0.00 | 0.00 | 0.00 |
| C15:0 | 0.21 | 0.27 | 0.28 | 0.39 | 0.37 | 0.44 | 0.23 | 0.28 | 0.30 | 0.29 | 0.25 | 0.26 |
| C16:0 | 7.22 | 7.37 | 10.45 | 9.15 | 8.33 | 8.60 | 6.98 | 7.75 | 7.71 | 7.50 | 7.20 | 7.36 |
| **C16:1 (PA)** | **0.07** | **0.09** | **0.62** | **0.27** | **0.24** | **0.35** | **0.08** | **0.17** | **0.08** | **0.08** | **0.04** | **0.31** |
| C17:0 | 0.11 | 0.20 | 0.22 | 0.26 | 0.23 | 0.25 | 0.16 | 0.20 | 0.22 | 0.19 | 0.12 | 0.10 |
| C17:1 | 0.11 | 0.33 | 0.00 | 0.33 | 0.33 | 0.32 | 0.29 | 0.32 | 0.34 | 0.31 | 0.00 | 0.00 |
| C18:0 | 0.33 | 0.47 | 0.77 | 0.46 | 0.44 | 0.45 | 0.59 | 0.51 | 0.50 | 0.62 | 0.28 | 0.29 |
| C18:1 n-7 | 0.06 | 0.00 | 2.08 | 0.00 | 0.00 | 0.00 | 0.00 | 0.00 | 0.00 | 0.00 | 0.04 | 0.33 |
| C18:1 n-9 | 0.04 | 4.96 | 2.15 | 0.92 | 0.93 | 0.97 | 10.24 | 5.38 | 1.17 | 2.56 | 0.00 | 0.11 |
| C18:2 n-6 | 0.00 | 1.51 | 0.35 | 0.16 | 0.16 | 0.20 | 0.60 | 1.22 | 2.02 | 8.30 | 0.00 | 0.00 |
| C18:3n3 | 0.23 | 0.89 | 0.34 | 0.30 | 0.28 | 0.30 | 0.25 | 0.31 | 0.54 | 1.85 | 0.25 | 0.21 |
| C18:3n6 | 0.11 | 0.12 | 0.13 | 0.12 | 0.12 | 0.14 | 0.11 | 0.12 | 0.13 | 0.11 | 0.12 | 0.11 |
| C18:4n3 | 0.08 | 0.22 | 0.61 | 0.32 | 0.32 | 0.35 | 0.20 | 0.22 | 0.25 | 0.20 | 0.12 | 0.00 |
| C20:0 | 0.09 | 0.12 | 0.13 | 0.09 | 0.09 | 0.09 | 0.10 | 0.10 | 0.10 | 0.10 | 0.03 | 0.06 |
| C20:3n3 | 0.00 | 0.00 | 0.00 | 0.00 | 0.00 | 0.00 | 0.00 | 0.00 | 0.00 | 0.00 | 0.00 | 0.00 |
| C20:3n6 | 0.21 | 0.20 | 0.17 | 0.20 | 0.18 | 0.18 | 0.18 | 0.20 | 0.21 | 0.18 | 0.22 | 0.21 |
| C20:4n3 | 0.82 | 0.75 | 0.77 | 0.76 | 0.77 | 0.78 | 0.71 | 0.76 | 0.78 | 0.69 | 0.84 | 0.83 |
| C20:4n6 | 0.16 | 0.18 | 0.56 | 0.20 | 0.20 | 0.22 | 0.17 | 0.19 | 0.21 | 0.18 | 0.18 | 0.15 |
| **C20:5 n-3 (EPA)** | **0.62** | **0.76** | **5.21** | **1.00** | **1.18** | **1.48** | **0.71** | **0.74** | **0.79** | **0.80** | **1.38** | **0.67** |
| C22:0 | 0.00 | 0.00 | 0.00 | 0.00 | 0.00 | 0.03 | 0.00 | 0.00 | 0.00 | 0.00 | 0.00 | 0.00 |
| C22:1n9 | 0.00 | 0.00 | 0.00 | 0.00 | 0.00 | 0.00 | 0.00 | 0.00 | 0.00 | 0.00 | 0.00 | 0.00 |
| C22:4n6 | 0.00 | 0.00 | 0.00 | 0.00 | 0.00 | 0.00 | 0.00 | 0.00 | 0.00 | 0.00 | 0.00 | 0.00 |
| C22:5n3 | 0.27 | 0.29 | 0.70 | 0.42 | 0.39 | 0.40 | 0.28 | 0.32 | 0.34 | 0.28 | 0.35 | 0.32 |
| C22:5n6 | 15.95 | 14.73 | 11.90 | 14.93 | 15.03 | 14.88 | 14.05 | 14.54 | 15.18 | 13.57 | 15.88 | 15.55 |
| **C22:6n3 (DHA)** | **70.96** | **64.52** | **59.04** | **67.15** | **67.97** | **67.17** | **62.21** | **64.87** | **67.26** | **60.16** | **70.68** | **70.07** |
| C24:0 | 0.07 | 0.00 | 0.12 | 0.00 | 0.00 | 0.00 | 0.00 | 0.00 | 0.00 | 0.00 | 0.10 | 0.11 |
| C24:1 | 0.08 | 0.10 | 0.04 | 0.04 | 0.03 | 0.08 | 0.00 | 0.05 | 0.09 | 0.08 | 0.04 | 0.08 |
| ND | 1.89 | 1.73 | 2.54 | 1.82 | 1.72 | 1.76 | 1.65 | 1.55 | 1.60 | 1.74 | 1.66 | 2.17 |

| | | | | | | | | | | | | |
|---|---|---|---|---|---|---|---|---|---|---|---|---|
| FFA distillate (Free Fatty Acids distillate); FOEE (Fish Oil Esterified); FO (Fish Oil); HOSO (High Oleic Sunflower Oil); EPA-EE (EPA ethyl esters) | | | | | | | | | | | | |

**Table 6: Results obtained for Aurantiochytrium mangrovei CCAP4062/3**

| | *Aurantiochytrium mangrovei* CCAP4062/3 | | | | | | | | | | |
|---|---|---|---|---|---|---|---|---|---|---|---|
| | Control | Colza | FFA distillate | FOEE | FO 18% | FO crude | Flaxseed | HOSO | Olive | Soybean | Walnut |
| C12:0 | 0 | 0.07 | 0.06 | 0.09 | 0.09 | 0.09 | 0.06 | 0.03 | 0.07 | 0.07 | 0.08 |
| C14:0 | 2.57 | 2.22 | 2.54 | 3.30 | 3.36 | 3.43 | 2.20 | 2.24 | 2.45 | 2.42 | 2.51 |
| C14:1 | 0 | 0 | 0 | 0 | 0 | 0 | 0 | 0 | 0 | 0 | 0 |
| C15:0 | 0.95 | 0.79 | 0.80 | 1.08 | 1.00 | 1.16 | 0.84 | 0.73 | 0.85 | 0.89 | 1.13 |
| C16:0 | 34.85 | 32.08 | 29.20 | 34.29 | 34.07 | 34.09 | 30.74 | 33.25 | 33.64 | 34.33 | 32.12 |
| **C16:1 (PA)** | **0** | **0.07** | **0.50** | **0.54** | **0.55** | **0.61** | **0.07** | **0.04** | **0.12** | **0.06** | **0.04** |
| C17:0 | 0.34 | 0.31 | 0.31 | 0.40 | 0.34 | 0.34 | 0.31 | 0.30 | 0.31 | 0.33 | 0.37 |
| C17:1 | 0.17 | 0.15 | 0.17 | 0.16 | 0.16 | 0.16 | 0.17 | 0.14 | 0.15 | 0.16 | 0.16 |
| C18:0 | 0.95 | 1.00 | 1.15 | 1.05 | 1.08 | 1.08 | 1.22 | 1.08 | 1.05 | 1.11 | 1.05 |
| C18:1n7 | 0.15 | 0.37 | 2.41 | 0.92 | 1.13 | 0.88 | 0.24 | 0.21 | 0.36 | 0.23 | 0.24 |
| C18:1n9 | 0 | 4.21 | 2.23 | 0.80 | 0.93 | 0.49 | 2.40 | 6.28 | 4.22 | 1.09 | 1.52 |
| C18:2n6 | 0 | 1.33 | 0.36 | 0.11 | 0.17 | 0.09 | 1.90 | 0.38 | 0.95 | 2.02 | 5.64 |
| C18:3n3 | 0.17 | 0.71 | 0.33 | 0.20 | 0.23 | 0.18 | 6.64 | 0.16 | 0.20 | 0.41 | 1.33 |
| C18:3n6 | 0 | 0.08 | 0.10 | 0.10 | 0.09 | 0.10 | 0.07 | 0.07 | 0.08 | 0.08 | 0.08 |
| C18:4n3 | 0.16 | 0.14 | 0.66 | 0.30 | 0.35 | 0.29 | 0.15 | 0.14 | 0.15 | 0.15 | 0.16 |
| C20:0 | 0.20 | 0.24 | 0.27 | 0.22 | 0.24 | 0.25 | 0.24 | 0.23 | 0.23 | 0.22 | 0.21 |
| C20:3n3 | 0 | 0 | 0 | 0 | 0 | 0 | 0 | 0 | 0 | 0 | 0 |
| C20:3n6 | 0.10 | 0.11 | 0.10 | 0.11 | 0.10 | 0.11 | 0.10 | 0.11 | 0.11 | 0.12 | 0.10 |
| C20:4n3 | 0.51 | 0.51 | 0.58 | 0.49 | 0.49 | 0.55 | 0.49 | 0.50 | 0.49 | 0.50 | 0.48 |
| C20:4n6 | 0.11 | 0.12 | 0.51 | 0.13 | 0.15 | 0.18 | 0.11 | 0.12 | 0.14 | 0.14 | 0.12 |
| **C20:5n3 (EPA)** | **0.59** | **0.58** | **5.71** | **0.90** | **1.30** | **1.60** | **0.54** | **0.61** | **0.62** | **0.56** | **0.62** |
| C22:0 | 0 | 0.10 | 0 | 0.08 | 0.09 | 0.09 | 0.10 | 0.13 | 0.09 | 0.14 | 0.08 |
| C22:1n9 | 0 | 0 | 0 | 0 | 0 | 0 | 0 | 0 | 0 | 0 | 0 |
| C22:4n6 | 0 | 0 | 0 | 0 | 0 | 0 | 0 | 0 | 0 | 0 | 0 |
| C22:5n3 | 0.17 | 0.16 | 0.73 | 0.23 | 0.26 | 0.27 | 0.17 | 0.16 | 0.17 | 0.18 | 0.17 |
| C22:5n6 | 9.95 | 9.46 | 7.38 | 9.34 | 9.18 | 9.37 | 8.87 | 9.16 | 9.28 | 9.53 | 8.99 |
| **C22:6n3 (DHA)** | **47.07** | **43.94** | **41.11** | **43.48** | **42.93** | **42.97** | **41.28** | **42.70** | **43.18** | **44.19** | **41.85** |
| C24:0 | 0 | 0 | 0 | 0 | 0 | 0 | 0 | 0 | 0 | 0 | 0 |
| C24:1 | 0 | 0 | 0 | 0 | 0 | 0 | 0 | 0 | 0 | 0 | 0 |
| ND | 0.99 | 1.32 | 2.85 | 1.76 | 1.77 | 1.68 | 1.15 | 1.29 | 1.15 | 1.13 | 1.01 |

| | | | | | | | | | | | |
|---|---|---|---|---|---|---|---|---|---|---|---|
| FFA distillate (Free Fatty Acids distillate); FOEE (Fish Oil Esterified); FO (Fish Oil); HOSO (High Oleic Sunflower Oil). | | | | | | | | | | | |

The results presented in Tables 5 and 6 show that a change in fatty acids (FA) profile is observed in the extracted oil produced by both *Aurantiochytrium* strains when an oil is added to the medium as carbon source. Oils derived from fish and containing EPA allow for the enrichment of EPA in the strain where they are used. However, not all of them allow for an equivalent increase. Notably, despite a much higher initial EPA content, the use of EPA-EE is much less effective than FFA distillate for EPA build-up by the strain. FFA distillate allows to produce oils with more than 5% EPA, 30% DHA and 0.1% PA relative to the total fatty acids content.

### EXAMPLE 3: Comparison of integration of different oils used as carbon source by Aurantiochytrium and Schizochytrium strains

Two strains of different genus and species (*Aurantiochytrium mangrovei* CCAP4062/7 and *Schizochytrium limacinum SR21)* were cultivated as described in Example 1 but with different oils as carbon source (1% v/v of each tested oil with respect of the total weight of the fermentation broth). Then, the cells were harvested as described in Example 1 and analyzed.

For each fatty acid, the integration percentages were calculated as follow: $Integration percentages = \frac{FA content produced in presence of oil - FA content produced without oil}{FA content added in the medium}$ the FA contents being expressed in g/L of fermentation broth.

Accordingly, a negative value is observed when a fatty acid is produced at a lower content within the strain grown with oil in comparison to the strain grown without. Such a negative value shows a decrease in the fatty acid content in comparison to the control. Integration results are presented in the following Table 7.

| Integration % | *Aurantiochytrium mangrovei* CCAP4062/7 | | | *Schizochytrium limacinum SR21* | | |
|---|---|---|---|---|---|---|
| | FFA distillate | FO crude | FOEE | FFA distillate | FO crude | FOEE |
| C12:0 | 0 | 0 | 0 | 0 | 0 | 0 |
| C14:0 | 10.08 | 2.52 | 2.31 | 1.84 | 2.48 | 0.61 |
| C14:1 | 0 | 0 | 0 | 0 | 0 | 0 |
| C15:0 | 16.00 | 0 | 0 | 4.70 | 0 | 0 |
| C16:0 | 19.87 | 2.53 | 5.51 | -14.19 | -4.67 | -1.96 |
| **C16:1 (PA)** | **7.31** | **1.77** | **1.02** | **4.93** | **2.32** | **0.42** |
| C17:0 | 11.57 | 5.97 | 5.08 | 2.75 | -0.16 | 0.20 |
| C17:1 | 0 | 0 | 0 | 0 | 0 | 0 |
| C18:0 | 9.49 | 1.16 | 3.33 | -0.02 | -0.25 | 0.18 |
| C18:1n7 | 60.27 | -1.07 | -1.38 | 7.65 | 5.52 | 2.46 |
| C18:1n9 | 19.42 | 4.80 | 7.25 | 5.99 | 1.26 | 1.09 |
| C18:2n6 | 25.60 | 8.42 | 9.76 | 7.77 | 2.31 | 0.82 |
| C18:3n3 | 18.04 | 3.92 | 7.80 | 7.49 | 3.16 | 1.02 |
| C18:3n6 | 0 | 2.03 | 0 | 0 | 0 | 0 |
| C18:4n3 | 24.02 | 4.60 | 5.65 | 5.55 | 1.47 | 0.56 |
| C20:0 | 0 | -0.20 | 0 | 0 | 0.77 | 0 |
| C20:3n3 | 0 | 0 | 0 | 0 | 0 | 0 |
| C20:3n6 | 0 | 0 | 0 | 0 | 0 | 0 |
| C20:4n3 | 8.41 | -16.20 | 0 | 4.39 | 4.96 | 0 |
| C20:4n6 | 22.73 | 7.42 | 0 | 7.09 | 5.67 | 0 |
| **C20:5n3 (EPA)** | **22.10** | **5.84** | **6.48** | **7.43** | **6.17** | **0.18** |
| C22:0 | 0 | 0 | 0 | 0 | 0 | 0 |
| C22:1n9 | 0 | 0 | 0 | 0 | 0 | 0 |
| C22:4n6 | 0 | 0 | 0 | 0 | 0 | 0 |
| C22:5n3 | 0 | 0 | 0 | 0 | 0 | 0 |
| C22:5n6 | -256.63 | -766.39 | 0 | -112.74 | -176.46 | 0 |
| **C22:6n3 (DHA)** | **-5.84** | **-108.51** | **-81.16** | **-17.19** | **-35.74** | **-174.11** |
| C24:0 | 0 | 0 | 0 | 0 | 0 | 0 |
| C24:1 | 0 | 0 | 0 | 0 | 0 | 0 |
| ND | 9.91 | -2.62 | 0.15 | 3.10 | 1.83 | 1.23 |

The results thus obtained clearly show a difference in integration between FFA distillate, FO crude and FOEE. Unlike FFA distillate, FOEE and FO crude do not allow to produce an oil with a suitable fatty acid profile. Said substrates are not greatly integrated by strains of the genus *Aurantiochytrium* and *Schizochytrium.*

The nature of the oil used as carbon source is thus important and its choice results in a strongly differentiated integration.

### EXAMPLE 4: Comparison of fatty acid profiles of oils produced with different strains in presence of FFA distillate

Strains of different genus and species (*Aurantiochytrium mangrovei* CCAP4062/7, *Schizochytrium limacinum SR21* and *Auxenochlorella protohtecoides)* were cultivated as described in Example 1 in FFA distillate. Then, the cells were harvested as described in Example land their fatty acid profiles analyzed.

The percentages by weight of fatty acids present in the extracted oils are presented in the Table 8 here below. They are expressed relative to the total fatty acids content.

| Fatty acid (% in the biomass) | *Auxenochlorella protothecoides* | *Aurantiochytrium mangrovei CCAP4062*/*7* | *Schizochytrium limacinum SR21* |
|---|---|---|---|
| C12:0 | 0.06 | 0 | 0 |
| C14:0 | 2.16 | 0.87 | 3.12 |
| C14:1 | 0.02 | 0 | 0 |
| C15:0 | 0.22 | 0.28 | 0.65 |
| C16:0 | 10.40 | 10.45 | 30.33 |
| **C16:1 (PA)** | **2.67** | **0.62** | **2.65** |
| C17:0 | 0.20 | 0.22 | 0.51 |
| C17:1 | 0.18 | 0 | 0.39 |
| C18:0 | 2.26 | 0.77 | 1.95 |
| C18:1n7 | 0 | 2.08 | 1.79 |
| C18:1n9 | 38.95 | 2.15 | 4.54 |
| C18:2n6 | 20.47 | 0.35 | 0.74 |
| C18:3n3 | 1.60 | 0.34 | 0.41 |
| C18:3n6 | 0.15 | 0.13 | 0 |
| C18:4n3 | 1.29 | 0.61 | 0.97 |
| C20:0 | 0.36 | 0.13 | 0.44 |
| C20:3n3 | 0 | 0 | 0 |
| C20:3n6 | 0.05 | 0.17 | 0 |
| C20:4n3 | 0.28 | 0.77 | 0.63 |
| C20:4n6 | 0.62 | 0.56 | 1.08 |
| **C20:5n3 (EPA)** | **7.41** | **5.21** | **12.55** |
| C22:0 | 0 | 0 | 0 |
| C22:1n9 | 0 | 0 | 0 |
| C22:4n6 | 0 | 0 | 0 |
| C22:5n3 | 0.66 | 0.70 | 1.82 |
| C22:5n6 | 0.20 | 11.90 | 2.96 |
| **C22:6n3 (DHA)** | **5.15** | **59.04** | **29.29** |
| C24:0 | 0.15 | 0.12 | 0 |
| C24:1 | 0 | 0.04 | 0 |
| ND | 4.5 | 2.54 | 3.23 |

The results hereabove show that the strains of the genus *Aurantiochytrium* and *Schizochytrium* (which are both Thaustochytrids) allow to produce an oil with high EPA and DHA contents. On the contrary, *Auxenochlorella* strain, which is not a Thraustochytrid, produces an oil with only 5% of DHA in the fatty acid fraction.

### EXAMPLE 5: lab-scale production with different FFA distillate content

*Aurantiochytrium mangrovei* (CCAP4062/7) was cultured during 72 hours, between 25 and 30°C, in the suitable medium as previously described in Table 1, in heterotrophy, with 50g/L in the initial culture medium of glucose and with an additional 300g/L of glucose added through a feed syrup at 750 g/L (43%v/v) from 8 hours of culture up until 36 hours and with different amounts of FFA distillate injected in the culture medium after 36 hours of culture (56 g/L, 105g/L, 150g/L and 226 g/L). Then, the cells were harvested as described in Example 1. Both oils and biomasses were analyzed as described in Example 2 as well as for DCW and lipid content (%dry matter).

The percentages of fatty acids present in the harvested biomass are presented in Table 9 here below. They are expressed relative to the total FA content of the biomass.

| **Injected concentration (g/L)** | | **56** | **105** | **150** | **226** |
|---|---|---|---|---|---|
| Injected quantity (% v/v) | | 6 | 12 | 17 | 26 |
| DCW (g/L) | | 115.5 | 162.6 | 166.78 | 171.96 |
| Lipid content (%dry matter) | | 46.06 | 57.88 | 62.41 | 60.71 |
| Fatty acid profile (% in the biomass) | C14:0 | 1.63 | 2.18 | 3.22 | 3.81 |
| | C16:0 | 12.49 | 15.71 | 14.92 | 14.54 |
| | **C16:1 (PA)** | **1.66** | **1.87** | **2.46** | **2.83** |
| | C17:0 | 0.24 | 0.22 | 0.19 | 0.20 |
| | C17:1 | 0.38 | 0.18 | 0.55 | 0.60 |
| | C18:0 | 0.95 | 0.84 | 0.78 | 0.78 |
| | C18:1n7 | 2.37 | 3.20 | 2.45 | 2.19 |
| | C18:1n9 | 3.07 | 2.98 | 4.19 | 4.76 |
| | C18:2n6 | 0.47 | 0.58 | 0.73 | 0.77 |
| | C18:3n3 | 0.36 | 0.42 | 0.18 | 0.19 |
| | C18:3n6 | 0 | 0.15 | 0.54 | 0.62 |
| | C18:4n3 | 0.90 | 0.99 | 1.34 | 1.60 |
| | C20:0 | 0 | 0.16 | 0.16 | 0.16 |
| | C20:3n6 | 0.23 | 0.22 | 0.16 | 0.14 |
| | C20:4n3 | 0.80 | 0.87 | 0.88 | 0.87 |
| | C20:4n6 | 0.92 | 1.05 | 1.13 | 1.11 |
| | **C20:5n3 (EPA)** | **7.77** | **8.45** | **11.19** | **12.93** |
| | C22:0 | 0 | 0 | 0 | 0 |
| | C22:1n9 | 0 | 0 | 0 | 0 |
| | C22:5n3 | 1.21 | 1.09 | 7.81 | 7.04 |
| | C22:5n6 | 11.43 | 9.3 | 0.95 | 0.86 |
| | **C22:6n3 (DHA)** | **51.16** | **44.53** | **40.64** | **38.05** |
| | ND | 1.96 | 5.01 | 5.53 | 5.95 |

The percentages of fatty acids present in the extracted oils are presented in Table 10 and 11 here below. They are expressed relative to the total fatty acids content of the oil (table 10) as well as by weight in the oil (mg/g) (table 11).

**Table 10**

| **Injected concentration (g/L)** | | **150** | **226** |
|---|---|---|---|
| Fatty acid profile (% in the oil) | C14:0 | 3.49 | 4.17 |
| | C16:0 | 15.38 | 14.95 |
| | **C16:1 (PA)** | **2.78** | **3.27** |
| | C17:0 | 0.14 | 0.14 |
| | C17:1 | 0.18 | 0.17 |
| | C18:0 | 0.72 | 0.70 |
| | C18:1n9 | 4.46 | 5.14 |
| | C18:1n7 | 2.62 | 2.35 |
| | C18:2n6 | 0.69 | 0.76 |
| | C18:3n3 | 0.17 | 0.19 |
| | C18:3n6 | 0.57 | 0.66 |
| | C18:4n3 | 1.37 | 1.66 |
| | C20:0 | 0.14 | 0.13 |
| | C20:3n6 | 0 | 0 |
| | C20:4n3 | 0.94 | 0.97 |
| | C20:4n6 | 1.42 | 1.46 |
| | **C20:5n3 (EPA)** | **11.35** | **13.32** |
| | C22:0 | 0 | 0 |
| | C22:1n9 | 0 | 0 |
| | C22:5n3 | 8.00 | 7.12 |
| | C22:5n6 | 0.99 | 0.90 |
| | **C22:6n3 (DHA)** | **39.82** | **37.47** |
| | ND | 4.77 | 4.47 |

**Table 11**

| **Injected concentration (g/L)** | | **150** | **226** |
|---|---|---|---|
| fatty acids content (mg/g) as FA in the oil | C10:0 | 0.00 | 0.00 |
| | C11:0 | 0.00 | 0.00 |
| | C12:0 | 0.00 | 0.00 |
| | C13:0 | 0.00 | 0.00 |
| | C14:0 | 28.50 | 33.04 |
| | C14:1 | 0.00 | 0.00 |
| | C15:0 | 2.92 | 3.22 |
| | C15:1 | 0.00 | 0.00 |
| | C16:0 | 123.55 | 116.36 |
| | **C16:1 (PA)** | **22.12** | **25.25** |
| | C16:2 | ND | ND |
| | C16:2n4 | ND | ND |
| | C16:3 | ND | ND |
| | C16:4 | ND | ND |
| | C17:0 | 1.13 | 1.05 |
| | C17:1 | 1.40 | 1.28 |
| | C18:0 | 5.67 | 5.35 |
| | C18:1 | ND | ND |
| | C18:1n7 | 35.12 | 39.16 |
| | C18:1n9 | 20.61 | 17.93 |
| | C18:1n9t | 0.00 | 0.00 |
| | C18:1n9c | 0.00 | 0.00 |
| | C18:1n11 | ND | ND |
| | C18:2 | ND | ND |
| | C18:2n6 | 5.41 | 5.71 |
| | C18:2n6t | 0.00 | 0.00 |
| | C18:2n6c | 0.00 | 0.00 |
| | C18:3 | ND | ND |
| | C18:3n3 | 4.86 | 5.50 |
| | C18:3n6 | 1.30 | 1.41 |
| | C18:4n3 | 11.71 | 13.79 |
| | C20:0 | 1.13 | 1.00 |
| | C20:1 | ND | ND |
| | C20:1n9 | 0.00 | 0.00 |
| | C20:2 | 0.00 | 0.00 |
| | C20:3n3 | 0.00 | 0.00 |
| | C20:3n6 | 0.00 | 0.00 |
| | C20:4n3 | 8.09 | 8.05 |
| | C20:4n6 | 12.24 | 12.12 |
| | **C20:5n3 (EPA)** | **97.43** | **110.72** |
| | C21:0 | 1.43 | 1.27 |
| | C22:0 | 0.00 | 0.00 |
| | C22:1 | ND | ND |
| | C22:1n9 | 0.00 | 0.00 |
| | C22:2 | 0.73 | 0.81 |
| | C22:4n6 | ND | ND |
| | C22:5n3 | 8.51 | 7.55 |
| | C22:5n6 | 8.51 | 7.55 |
| | **C22:6n3 (DHA)** | **343.37** | **312.92** |
| | C23:0 | - | - |
| | C24:0 | 0.77 | 0.00 |
| | C24:1 | 0.00 | 0.00 |
| Oméga-3 Totaux (mg/g) as FA in the oil | | 474.09 | 458.65 |

The results presented hereabove in Tables 10 and 11 show that FFA distillate as carbon source can be added at any concentration in view of integrating fatty acids of interest. The higher the added oil content, the higher DCW, lipid content, EPA and PA contents but the lower DHA.

These results also show that FA percentage results in the biomass and in the oil are similar.

### EXAMPLE 6: Lab-scale production with FFA distillate added at different cultivation time

*Aurantiochytrium mangrovei* (CCAP4062/7) was cultured during around 85 hours between 25 and 30°C, in the suitable medium as previously described in Table 1, in heterotrophy, with 50g/L in the initial culture medium of glucose and an additional 120g/L or 320g/L of glucose (10%v/v) from 8 hours of culture up until the addition of FFA distillates, with around 10% v/v of FFA distillate injected in the culture medium after 17 or 48 hours of culture. Then, the cells were harvested as described in Example 1 and analyzed for lipid content (%dry analysis) and fatty acids profiles in the biomass and in the oil.

The percentages of fatty acids present in the harvested biomass are presented in the Table 12 here below. They are expressed relative to the total fatty acid content of the biomass.

| | | | |
|---|---|---|---|
| Induction hour | | 17h | 48h |
| Injected quantity (%v/v) | | 10% | 11% |
| Injected concentration (g/L) | | 85 g/L | 85 g/L |
| Lipid content (%dry matter) | | 46.25 | 60.37 |
| Fatty acids (% in the biomass) | C14:0 | 3.42 | 2.28 |
| | C16:0 | 15.92 | 9.57 |
| | **C16:1 (PA)** | **2.77** | **2.27** |
| | C17:0 | 0.26 | 0.1 |
| | C17:1 | 0.13 | 0.28 |
| | C18:0 | 0.9 | 0.45 |
| | C18:1n7 | 6.7 | 1.6 |
| | C18:1n9 | 5.59 | 1.11 |
| | C18:2n6 | 1.06 | 0.4 |
| | C18:3n3 | 0.68 | 0.48 |
| | C18:3n6 | 0.19 | 0.18 |
| | C18:4n3 | 1.7 | 1.46 |
| | C20:0 | 0.19 | 0.09 |
| | C20:3n6 | 0.18 | 0.15 |
| | C20:4n3 | 0.8 | 0.76 |
| | C20:4n6 | 1.41 | 0.61 |
| | **C20:5n3 (EPA)** | **16.4** | **9.65** |
| | C22:0 | 0.12 | 0 |
| | C22:1n9 | 0 | 0 |
| | C22:5n3 | 1.06 | 0.5 |
| | C22:5n6 | 2.87 | 10.19 |
| | **C22:6n3 (DHA)** | **30.18** | **52.22** |
| | ND | 7.47 | 5.65 |

The results presented hereabove show that FFA distillate as carbon source can be added at any time during the cultivation in view of integrating fatty acids of interest. Interestingly, an addition at 17h allows a higher EPA and PA integration but a lower DCW yield whereas an addition at 48h allows a higher DCW yield but a lower EPA and PA integration.

### EXAMPLE 7: Pilot scale production

*Aurantiochytrium mangrovei* (CCAP4062/7) was cultured during around 85 hours as described in Example 5 but with 17% v/v (150g/L) of FFA distillate injected in the culture medium after 36 hours of culture, in a 1000L reactor. Then, the cells were harvested as described in Example 1 and analyzed.

Oxidation levels and fatty acids profiles of the oils produced by the strain as well as of the FFA distillate used as carbon source and fatty acids profiles of the triglyceride fraction of produced oils were analyzed.

The percentages of fatty acids present in the harvested biomass are presented in the Table 13 here below. They are expressed relative to the total fatty acids content of the biomass.

| | | |
|---|---|---|
| DCW (g/L) | | 171.84 |
| Lipid content (%dry matter) | | 60.63 |
| Fatty acid (% in the biomass) | C12:0 | 0.09 |
| | C14:0 | 3.57 |
| | C14:1 | 0 |
| | C15:0 | 0.41 |
| | C16:0 | 19.28 |
| | **C16:1 (PA)** | **3.11** |
| | C17:1 | 0.16 |
| | C18:0 | 1.17 |
| | C18:1n7 | 4.77 |
| | C18:1n9 | 4.53 |
| | C18:2n6 | 1.00 |
| | C18:3n3 | 0.58 |
| | C18:3n6 | 0.17 |
| | C18:4n3 | 1.26 |
| | C20:0 | 0.17 |
| | C20:3n3 | 0.05 |
| | C20:3n6 | 0.20 |
| | C20:4n3 | 0.93 |
| | C20:4n6 | 1.31 |
| | **C20:5n3 (EPA)** | **10.82** |
| | C22:0 | 0 |
| | C22:1n9 | 0 |
| | C22:4n6 | ND |
| | C22:5n3 | 1.37 |
| | C22:5n6 | 5.85 |
| | **C22:6n3 (DHA)** | **33.1** |
| | C24:0 | 0.11 |
| | C24:1 | 0 |

The percentages of fatty acids present in the extracted oil are presented in the Table 14 here below. They are expressed relative to the total fatty acids content of the oil and in mg/g of oil.

| Fatty acids in the oil | % | mg/g |
|---|---|---|
| C10:0 | ND | 0.00 |
| C11:0 | ND | 0.00 |
| C12:0 | 0.00 | 0.00 |
| C13:0 | ND | 0.00 |
| C14:0 | 4.10 | 34.84 |
| C14:1 | 0.00 | 0.00 |
| C15:0 | 0.40 | 3.40 |
| C15:1 | ND | 0.00 |
| C16:0 | 21.35 | 178.05 |
| **C16:1 (PA)** | **3.69** | **30.58** |
| C17:0 | 0.28 | 2.28 |
| C17:1 | 0.00 | 0.00 |
| C18:0 | 1.18 | 9.71 |
| C18:1n9 | 4.98 | 43.95 |
| C18:1n7 | 5.38 | 40.67 |
| C18:1n9 | ND | 0.00 |
| C18:2n6 | 0.86 | 7.00 |
| C18:3n3 | 0.60 | 5.23 |
| C18:3n6 | 0.00 | 0.00 |
| C18:4n3 | 1.27 | 11.09 |
| C20:0 | 0.00 | 0.00 |
| C20:1n9 | ND | 1.41 |
| C20:2 | ND | 0.00 |
| C20:3n3 | 0.00 | 0.00 |
| C20:3n6 | 0.00 | 0.00 |
| C20:4n3 | 0.96 | 8.39 |
| C20:4n6 | 1.54 | 13.44 |
| **C20:5n3 (EPA)** | **11.21** | **99.33** |
| C21:0 | ND | 1.72 |
| C22:0 | 0.00 | 0.00 |
| C22:1n9 | 0.00 | 0.00 |
| C22:2 | ND | 0.00 |
| C22:5n3 | 1.45 | 12.68 |
| C22:5n6 | 5.49 | 12.68 |
| **C22:6n3 (DHA)** | **31.95** | **278.49** |
| C24:0 | 0.00 | 0.00 |
| C24:1 | 0.00 | 0.00 |
| Omega-3 Total | | 415.31 |

Glyceridic profile of the extracted oil was also analyzed and results are presented in the following Table 15, expressed in relative %.

| | |
|---|---|
| Glycerol | <0.1 |
| Free fatty acids | <0.1 |
| Monoglycerides | <0.1 |
| Sterols | 0.6 |
| Diglycerides | 0.6 |
| Esters de sterols | <0.1 |
| Triglycerides | 98.9 |
| Not identified | <0.1 |

Fatty acids profile of the triglyceride fractions was analyzed and results are presented in the following Table 16.

| FA content % in triglycerides | |
|---|---|
| C12:0 | 0.12 |
| C13:0 | <0.05 |
| C14:0 | 4.06 |
| C14:1 | 0.37 |
| C15:0 | 0.47 |
| C16:0 | 20.77 |
| **C16:1 (PA)** | **3.97** |
| C16:2 | 0.6 |
| C16:3 | 0.59 |
| C16:4 | 0.54 |
| C17:0 | 0.57 |
| C17:1 | 0.39 |
| C18:0 | 1.31 |
| C18:1 trans | 0.09 |
| C18:1 | 10.48 |
| C18:2trans | <0.05 |
| C18:2 cis | 1.62 |
| C18:3 (n-6) | 0.19 |
| C18:3 trans | <0.05 |
| C18:3 (n-3) cis | 0.68 |
| C18:4 (n-3) | <0.05 |
| C20:0 | 0.16 |
| C20:1 | 0.3 |
| C20:2 (n-6) | 1.34 |
| C20:3 (n-6) | 0.23 |
| C20:4 (n-6) | 1.31 |
| C20:3 (n-3) | 0.05 |
| C20:4 (n-3) | 0.99 |
| **C20:5 (n-3) (EPA)** | **10.65** |
| C22:0 | 0.07 |
| C22:1 | 0.24 |
| C22:4 (n-6) | 0.08 |
| C21:5 (n-3) | 0.35 |
| C22:5 (n-6) | 5.39 |
| C22:5 (n-3) | 1.76 |
| **C22:6 (n-3) (DHA)** | **30.07** |
| C24:0 | 0.05 |
| C24:1 | 0.05 |

In view of the above, the produced oil has a high content of triglycerides (around 99%) and DHA, EPA and PA are mainly in triglyceride form.

The oxidation results are presented in the following Table 17.

| | FFA distillate | extracted oil |
|---|---|---|
| Peroxide value (PV) - mEqO₂/kg | 0.25 | 1.40 |
| Para-Anisidine Value (pAV) | 113.7 | 14.80 |

The results presented hereabove in Table 17 show that even if FFA distillate used as a carbon source had a very high pAV value, the final oil produced by the strain has low PV and pAV.

These results thus show that the oil extracted by the process of the present invention is non-oxidized, which renders it suitable for human consumption.

### EXAMPLE 8: EPA from marine oil integration within triglycerides of the produced oil

*Aurantiochytrium mangrovei* CCAP4062/7strain was cultured within a culture medium as described hereabove in Table 1 with glucose (30g/L) and 10g/L of different marine oils carbon source (FFA distillate, FOEE, and FO crude) during 72 hours at 26°C.

Glucose consumption and lipid formation were followed during the whole culture of 72 hours (lipid content, %wet matter). Then, the cells were harvested as described in Example 1 and analyzed for DCW, lipid content %dry matter and FA profile in the biomass at t=0h and t=72h of culture were analyzed. Further, the localization of EPA was determined by analyzing EPA contents of different lipidic fractions after separation by thin layer chromatography and FA profile of FFA in the lipidic phase of the culture medium at the end of the culture t=72h.

The results are presented in the following Table 18 and Figures 1 to 7.

| **Condition** | **Control** | **FFA** | **FOEE** | **FO** |
|---|---|---|---|---|
| Culture time (h) | 72 | 72 | 72 | 72 |
| Lipids (% dry matter) | 40.21 ± 5.14 | 45.19 ± 4.17 | 40.36 ± 5.33 | 39.60 ± 3.18 |
| Biomass (g/L) | 15.05 ± 0.46 | 18.65 ± 1.06 | 16.36 ± 1.21 | 15.39 ± 0.01 |

As shown on Figure 2, during the first 24 hours of culture, barely significant lipids were produced by the strain under any condition. The 10% of lipids present in the beginning are the constituent lipids present mainly in the membrane of the cells. After this point between 24 and 48 hours, lipid concentration increased in the biomass. As seen in Figure 1, at 48 hours of culture, glucose was no longer available as a carbon source, thus the intracellular lipids content increased thanks to biointegration of the marine oils carbon sources.

As shown in the Figure 3, the condition without addition of an oily carbon source yielded the lowest biomass at 15.05 ± 0.46 g/L. According to these results, the conditions where the highest biomass was obtained is the condition were the FFA distillate was added to the media, yielding a biomass of 18.65 ± 1.06 g/L. The other two conditions cannot be described as significantly different to each other, and the FO conditions yields a biomass statistically similar to the control condition. Thus, these results clearly show that the best performance in terms of DCW are achieved when using FFA distillate.

The results presented in the Figures 4 and 5, comparing the conditions using FFA distillate and FOEE show the distribution of EPA in the three fractions analyzed: triglycerides (TG), free fatty acids (FA) and phospholipids (PL).

The presence of EPA in the phospholipid fraction of the cells demonstrates integration of the material into the cells' metabolism since the raw material contains only free fatty acids, ethyl esters and glycerides. Likewise, the presence of EPA in the triglyceride fraction of the produced oil is also strongly suggestive of integration into the cellular metabolism. According to these results, the addition of FFA into the culture media resulted in a 6.65-fold increase of EPA in the TG, compared to the FOEE condition.

As shown on figure 6, the fatty acid profile of the culture medium after 72h of culture of *Aurantiochytrium* strain in presence of FFA distillate, present a relative decrease in some FA, including myristic acid, oleic acid, and EPA as well as the increase in others. This modification of the fatty acid profile of the lipidic phase of the culture medium is clear evidence of action by the cells and may indicate preferential uptake of some fatty acids.

### EXAMPLE 9: Combination of glucose and marine oils during the culture of Aurantiochytrium mangrovei CCAP406217 strain

The *Aurantiochytrium mangrovei* CCAP4062/7strain was cultivated in several different conditions (in duplicate, n=2, results given are mean values), within a culture medium as described hereabove in Table *1* for *Aurantiochytrium,* with an inoculation rate at 1%v/v, in heterotrophy during 72h at 26°C and with or without glucose and with or without marine oils: FFA distillate, FOEE or FO crude. In particular, the strain was cultivated without glucose and without any marine oil (A), with 30g/L of glucose but without any marine oil (B), with 10 g/L of glucose and 10 g/L of FFA distillate (C) or of FOEE (D) or of FO crude (E), without glucose but with 15g/L FFA distillate (F) or of FOEE (G) or of FO crude (H). Growth of the strain was followed by measuring the optical density then, the cells were harvested according to Example 1 and analyzed for determining FA content and fat content.

Optical densities (600nm) in presence of glucose or not for each tested marine oils are presented in Figures 7a (without glucose) and 7b (with glucose).

As shown in figures 7a and 7b, the OD of the biomasses cultivated with marine oils without glucose is higher than the OD of the biomass without either glucose or marine oil (Figure 7a). Thus, the strain uses marine oils for growth. In the presence of glucose, marine oils do not interfere with cell growth as biomasses reach the same OD as the biomass cultivated with glucose only (Figure 7b). Further, it has to be noted that the culture medium of the control with glucose only (B), comprises 30g/L of glucose, whereas the culture media comprising glucose and marine oil comprise only 10g/L of glucose (C, D and E). However, each biomass reaches the same final OD. Accordingly, it can be concluded that the strain uses the marine oils as an alternative carbon source to glucose.

FA contents in the produced biomasses after the 72h of culture are presented in the following Tables 19 and 20. They correspond to average of two analyses and are expressed in % relative to the total FA content of the biomasses.

**Table 19: In absence of glucose**

| | **PA** (%) | **EPA (%)** | **DHA (%)** | **Lipid content (%dry matter)** |
|---|---|---|---|---|
| Control no glucose (A) | 20.0 ± 0.0 | 1.9 ± 0.0 | 62.5 ± 0.0 | 5.6 ± 0.0 |
| FFA distillate only (F) | 14.5 ± 0.2 | 21.3 ± 0.6 | 29.5 ± 0.4 | 29.6 ± 1.7 |
| FO crude only (G) | 23.6 ± 1.0 | 12.6 ± 0.3 | 14.2 ± 0.1 | 39.9 ± 0.6 |
| FOEE only (H) | 21.1 ± 3.5 | 7.4 ± 1.3 | 13.1 ± 1.4 | 38.0 ± 2.6 |

**Table 20: In presence of glucose**

| | **PA** (%) | **EPA (%)** | **DHA (%)** | **Lipid content (%dry matter)** |
|---|---|---|---|---|
| Control glucose only (B) | 9,1 ± 0.0 | 0.8 ± 0.0 | 70.8 ± 0.0 | 29.5 ± 0.0 |
| FFA distillate + glucose (C) | 16,8 ± 0.6 | 16.6 ± 0.8 | 33.7 ± 1.0 | 23.9 ± 0.4 |
| FO crude + glucose (D) | 18,5 ± 1.3 | 9.6 ± 1.6 | 26.9 ± 2.9 | 22.2 ± 7.4 |
| FOEE + glucose (E) | 17,9 ± 2.9 | 4.4 ± 2.0 | 30.4 ± 1.1 | 22.5 ± 4.6 |

The results hereabove clearly show that the biomasses cultivated with a marine oil but without glucose have higher lipids contents than biomasses cultivated without either glucose or marine oil and with glucose only. Cells contained more EPA and less DHA in the presence of marine oils.

## Claims

1. A crude microbial oil of at least one Thraustochytrid comprising:
- at least 94% of triglycerides relative to the total lipids,
- more than 5% by weight of eicosapentaenoic acid (EPA), relative to the total weight of oil,
- at least 27% by weight, preferably at least 30% by weight of docosahexaenoic acid (DHA), relative to the total weight of oil, and
- from 0.1% to 5% by weight of palmitoleic acid (PA), relative to the total weight of oil;
wherein the p-anisidine value of said oil being lower than or equal to 20.

2. The crude microbial oil according to claim 1, wherein said oil comprises at least 35% by weight of omega-3 fatty acids, preferably from 35% to 90% by weight, and more preferably from 36% to 50% by weight, relative to the total weight of oil.

3. The crude microbial oil according to claim **1** or **2,** wherein the amount of EPA ranges from 5% to 30% by weight, preferably from 7% to 20% by weight, relative to the total weight of said oil.

4. The crude microbial oil according to any one of claims **1** to **3,** wherein the amount of EPA ranges from 5% to 30%, preferably from 7% to 20%, relative to the total triglycerides content.

5. The crude microbial oil according to any one of claims **1** to **4,** wherein the amount of DHA ranges from 30% to 60%, preferably from 40% to 50%, relative to the total the triglycerides content.

6. The crude microbial oil according to any one of claims **1** to **5,** wherein the Thraustochytrid is selected from strains of the genera *Aurantiochytrium* and *Schizochytrium,* preferably from strains of *Aurantiochytrium mangrovei* species and *Schizochytrium limacinum* species, more preferably from the strains *Aurantiochytrium mangrovei CCAP 4062*/*7; Aurantiochytrium mangrovei CCAP 4062*/*2; Aurantiochytrium mangrovei CCAP 4062*/*); Aurantiochytrium mangrovei CCAP 4062*/*4; Aurantiochytrium mangrovei CCAP 4062*/*5; Aurantiochytrium mangrovei CCAP 4062*/*6; Aurantiochytrium mangrovei CCAP 4062*/*1; Schizochytrium limacinum SR21 (ATCC MYA-1381)*; and even more preferably selected from the strains *Aurantiochytrium mangrovei CCAP4062*/*7* and *CCAP4062*/*3* and *Schizochytrium limacinum SR21.*

7. A process for producing a crude microbial oil of Thraustochytrid comprising the following steps:
a) culturing a Thraustochytrid strain within a culture medium in a reactor to form a biomass comprising an oil,
b) recovering the biomass formed at step a), and
c) extracting the oil from the biomass recovered at step b);
wherein, in step a), the thraustochytrid strain is cultured in presence of a marine oil comprising at least 50% by weight of free fatty acids, relative to the total weight of said marine oil, preferably an oxidized marine oil, more preferably an oxidized oil derived from fish.

8. The process according to claim **7,** wherein the marine oil comprises from about 50% to 99% by weight, preferably from about 60 to 95% by weight of free fatty acids, relative to the total weight of the marine oil.

9. The process according to claim **7** or **8,** wherein the marine oil comprises at least 5%, preferably from 5% to 55%, and more preferably from 8% to 40%, of EPA in its free fatty acid form, relative to the total fatty acids content of the marine oil.

10. The process according to any one of claims **7** to **9,** wherein the marine oil is present in the initial culture medium of the culturing step a) or the marine oil is added in the culture medium during the culturing step a), preferably after at least about 5 hours of culture, more preferably after about 15 hours of culture, still more preferably after at least about 45 hours of culture, optionally after nitrogen starvation of the biomass.

11. The process according to any one of claims **7** to **10,** wherein the marine oil comprises at least 0.1%, preferably from 0.1% to 60%, and more preferably from 10% to 50%, of PA in its free fatty acid form, relative to the total fatty acids content of the marine oil.

12. The process according to any one of claims **7** to **11,** wherein the marine oil comprises at least 1%, preferably from 1% to 20%, more preferably from 5% to 15%, of DHA in its free fatty acid form, relative to the total fatty acids content of the marine oil.

13. The process according to any one of claims **7** to **12,** wherein the Thraustochytrids is selected from strains of the genera *Aurantiochytrium* and *Schizochytrium,* preferably from strains of *Aurantiochytrium mangrovei* species and *Schizochytrium limacinum* species, more preferably from the strains *Aurantiochytrium mangrovei CCAP 4062*/*7; Aurantiochytrium mangrovei CCAP 4062*/*2; Aurantiochytrium mangrovei CCAP 4062*/*); Aurantiochytrium mangrovei CCAP 4062*/*4; Aurantiochytrium mangrovei CCAP 4062*/*5; Aurantiochytrium mangrovei CCAP 4062*/*6; Aurantiochytrium mangrovei CCAP 4062*/*1; Schizochytrium limacinum SR21 (ATCC MYA-1381)*; and even more preferably selected from the strains *Aurantiochytrium mangrovei CCAP4062*/*7* and *CCAP4062*/*3* and *Schizochytrium limacinum SR21.*

14. The process according to any one of claims **7** to **13,** wherein the crude microbial oil extracted at step c) is a non-oxidized oil.

15. A crude microbial oil of Thraustochytrid obtained by the process according to any one of claims **7** to **14,** wherein the p-anisidine value of said oil being lower than or equal to 20, preferably lower than or equal to 15.

16. A biomass of Thraustochytrid comprising a crude microbial oil according to any one of claims **1** to **6** and **15.**
